# EUROPÄISCHE PATENTSCHRIFT

(11) **EP 0 967 980 B1**
(45) Veröffentlichungstag und Bekanntmachung des Hinweises auf die Patenterteilung: **02.05.2003**
(21) Anmeldenummer: 98902979.8
(22) Anmeldetag: 07.01.1998
(51) Int. Cl.: A61K 31/505, A61P 17/02

(54) **VERWENDUNG VON AMINOTHIAZOLEN ZUR WUND- UND HAUTBEHANDLUNG**
USE OF AMINOTHIAZOLES FOR TREATING WOUNDS AND SKIN
UTILISATION D'AMINOTHIAZOLES DANS LE TRAITEMENT DE PLAIES OU DE LA PEAU

(30) Priorität: 13.01.1997 DE 19700795
(43) Veröffentlichungstag der Anmeldung: 05.01.2000
(73) Patentinhaber: BaFuS Marketing GmbH, 40878 Ratingen (DE)
(72) Erfinder: SCHALLER, Klaus, D-42109 Wuppertal (DE); BAASNER, Bernd, D-51467 Bergisch Gladbach (DE); LISZKAY, Magdalena, D-42113 Wuppertal (DE); WERLING, Hans-Otto, D-42113 Wuppertal (DE); PROKSCH, Ehrhardt, D-24113 Molfsee (DE)
(74) Vertreter: Hofmeister, Frank Horst
(86) Internationale Anmeldenummer: EP9800033
(87) Internationale Veröffentlichungsnummer: WO98030212

(56) Entgegenhaltungen:
- EP-A- 0 365 915
- EP-A- 0 718 296
- DE-A- 3 836 161

## Beschreibung

Die Erfindung betrifft die Verwendung von substituierten 2-Aminothiazolen zur Verbesserung der Wundheilung sowie die Verwendung dieser Verbindungen zur Herstellung von entsprechenden Arzneimitteln.

Aus EP-0 365 915 ist bereits bekannt, daß solche Verbindungen antimikrobielle, insbesondere stark antibakterielle und antimykotische Wirkung besitzen.

Überraschenderweise wurde nun gefunden, daß diese Verbindungen auch eine deutliche Verbesserung der Wundheilung bewirken.

Die Erfindung betrifft daher die Verwendung von 2-Aminothiazolen der allgemeinen Formel (I), 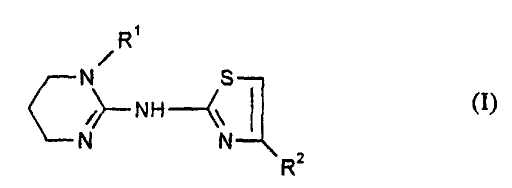 in welcher
- R¹: für Wasserstoff oder für geradkettiges oder verzweigtes Alkyl mit 1 bis 4 Kohlenstoffatomen steht und
- R²: für einen Rest der Formel 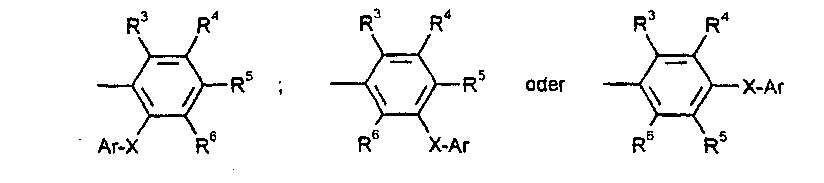 steht,
wobei
- R³, R⁴, R⁵ und R⁶: unabhängig voneinander jeweils für Wasserstoff, Fluor, Chlor, Brom, Iod, Nitro, für jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 4 Kohlenstoffatomen in den jeweiligen Alkylteilen oder für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen - insbesondere Fluor, Chlor, Brom oder Iod - stehen,
- X: für Sauerstoff, Schwefel, Sulfinyl oder Sulfonyl steht und
- Ar: für jeweils gegebenenfalls einfach oder mehrfach, gleich oder verschieden substituiertes Phenyl, α-Naphthyl, β-Naphthyl, Tetrahydronaphthyl oder Indanyl steht, wobei als Substituenten jeweils in Frage kommen: Fluor, Chlor, Brom, Iod, jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 8 Kohlenstoffatomen in den jeweiligen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, Cycloalkyl mit 3 bis 7 Kohlenstoffatomen, Phenylalkyl oder Phenoxyalkyl mit jeweils 1 bis 4 Kohlenstoffatomen im geradkettigen oder verzweigten Alkylteil sowie Phenyl oder Phenoxy,
sowie von deren physiologisch verträglichen Säureadditionssalzen zur Verbesserung der Wundheilung.

Die Verbindungen der Formel (I) stehen im Gleichgewicht mit den tautomeren Verbindungen der Formeln (Ia) und (Ib), 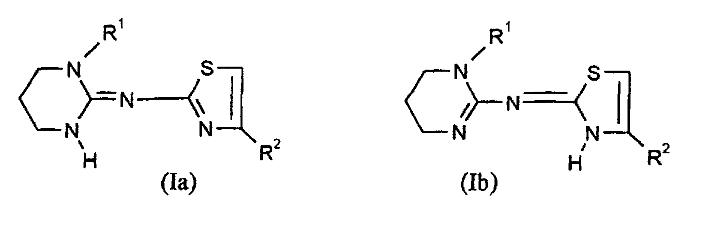 (wobei R¹ und R² jeweils die oben angegebene Bedeutung haben,) welche ebenfalls erfindungsgemäß beansprucht werden.

Ein Verfahren zur Herstellung der obengenannten Verbindungen ist in EP-0 365 915 beschrieben.

Die erfindungsgemäß substituierten 2-Aminothiazole sind durch die Formel (I) allgemein definiert. Bevorzugt ist die erfindungsgemäße Verwendung von Verbindungen der Formel (I), bei welchen
- R¹: für Wasserstoff, Methyl oder Ethyl steht,
- R²: für einen Rest der Formel 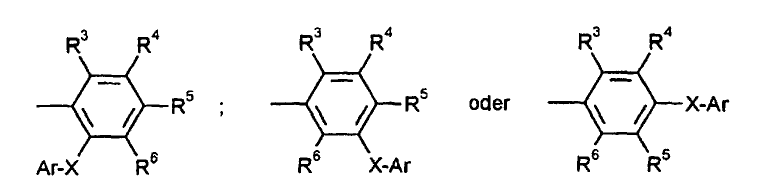 steht,
wobei
- R³, R⁴, R⁵ und R⁶: unabhängig voneinander jeweils für Wasserstoff, Fluor, Chlor, Brom, Nitro, Methyl, Ethyl, Methoxy, Ethoxy, Methoxycarbonyl, Ethoxycarbonyl, Dimethylamino, Diethylamino, Methylthio, Ethylthio, Methylsulfinyl, Ethylsulfinyl, Methylsulfonyl oder Ethylsulfonyl oder für Halogenmethyl, Halogenethyl, Halogenmethoxy, Halogenethoxy, Halogenmethylthio, Halogenethylthio, Halogenmethylsulfinyl, Halogenethylsulfinyl, Halogenmethylsulfonyl oder Halogenethylsulfonyl mit jeweils 1 bis 5 gleichen oder verschiedenen Halogenatomen, insbesondere Fluor, Chlor oder Brom stehen,
- X: für Sauerstoff, Schwefel, Sulfinyl oder Sulfonyl steht und
- Ar: für jeweils gegebenenfalls ein- bis fünffach gleich oder verschieden substituiertes Phenyl, α-Naphthyl, β-Naphthyl, Tetrahydronaphthyl oder Indanyl steht, wobei als Substituenten jeweils in Frage kommen: Fluor, Ghlor, Brom, Nitro, jeweils geradkettiges oder verzweigtes Alkyl, Alkoxy, Alkoxycarbonyl oder Dialkylamino mit jeweils 1 bis 6 Kohlenstoffatomen in den jeweiligen Alkylteilen, jeweils geradkettiges oder verzweigtes Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, insbesondere Fluor, Chlor oder Brom, Cyclohexyl mit 3 bis 6 Kohlenstoffatomen, Phenylalkyl oder Phenoxyalkyl mit jeweils 1 bis 3 Kohlenstoffatomen im geradkettiges oder verzweigten Alkylteil sowie Phenyl oder Phenoxy.

Besonders bevorzugt ist die erfindungsgemäße Verwendung von Verbindungen der Formel (I), bei welchen
- R¹: für Wasserstoff oder Methyl steht und
- R²: für einen Rest 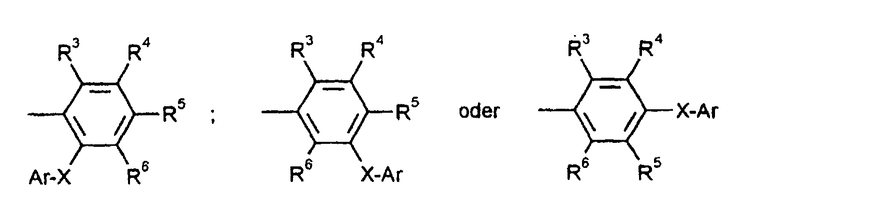 steht,
wobei
- R³, R⁴, R⁵ und R⁶: unabhängig voneinander jeweils für Wasserstoff, Fluor, Chlor, Nitro, Methyl, Methoxy, Ethoxycarbonyl, Methoxycarbonyl, Dimethylamino, Methylthio, Trifluormethyl, Trifluormethoxy, Trifluormethylthio, Trifluormethylsulfinyl oder Trifluormethylsulfonyl stehen,
- X: für Sauerstoff oder Schwefel stehen und
- Ar: für jeweils gegebenenfalls ein- bis vierfach, gleich oder verschieden substituiertes Phenyl, α-Naphthyl, β-Naphthyl, Tetrahydronaphthyl oder Indanyl steht, wobei als Substituenten jeweils in Frage kommen: Fluor, Chlor, Brom, Nitro, Methyl, Ethyl, n- oder i-Propyl, n-, i-, s- oder t-Butyl, Methoxy, Ethoxy, n- oder i-Propoxy, Methoxycarbonyl, Ethoxycarbonyl, Dimethylamino, Diethylamino, Methylthio, Ethylthio, Methylsulfinyl, Methylsulfonyl, Trifluormethyl, Trifluormethoxy, Trifluormethylthio, Trifluormethylsulfinyl, Trifluormethylsulfonyl, Cyclopentyl, Cyclohexyl, Benzyl, Phenylethyl, Phenylpropyl, Phenoxymethyl, Phenyl oder Phenoxy.

Besonders bevorzugte Verbindungen sind auch Additionsprodukte aus Säuren und denjenigen substituierten 2-Aminothiazolen der Formel (I), in denen die Substituenten R¹ und R² die Bedeutungen haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Oxalsäure, Maleinsäure, Malonsäure, Bernsteinsäure, Fumarsäure, Glutarsäure, Hydroxyglutarsäure, Adipinsäure, Oleinsäure, Weinsäure, Äpfelsäure, Zitronensäure, Benzoesäure, Salicylsäure, Sorbinsäure und Milchsäure, Sulfonsäuren, wie z.B. Methansulfonsäure, p-Chlorbenzolsulfonsäure, p-Toluolsulfonsäure und 1,5-Naphthalindisulfonsäure, Schwefelsäurehalbester wie Schwefelsäuremonomethylester oder Schwefelsäuremonoethylester sowie Saccharin oder Thiosaccharin.

Insbesondere bevorzugt ist die erfindungsgemäße Verwendung von Verbindungen der Formel (I), bei welchen
- R¹: für Wasserstoff steht und
- R²: für einen Rest der Formel 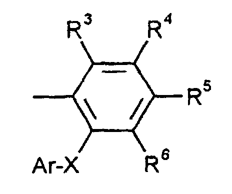 steht,
wobei
- R³, R⁴, R⁵ und R⁶: unabhängig voneinander jeweils für Wasserstoff, Methyl oder Nitro stehen,
- X: für Sauerstoff steht und
- Ar: für gegebenenfalls ein- bis dreifach, gleich oder verschieden substituiertes Phenyl steht, wobei als Substituenten insbesondere in Frage kommen: Nitro, Methyl, Ethyl, n- oder i-Propyl, n-, i-, s- oder t-Butyl, Methoxy, Methylthio, Cyclopentyl, Cyclohexyl, Phenyl oder Phenoxy.

Die erfindungsgemäßen Verbindungen der Formel (I) und ihre Säureadditionssalze zeichnen sich durch eine ausgesprochen günstige Wirkung auf den Verlauf der Wundheilung aus. Insbesondere wird die Wundheilung und die Reparatur der Permeabilitätsbarriere der Haut beschleunigt. Sie können daher zur Wund- und Hautbehandlung bei Menschen und Tieren eingesetzt werden.

Als Indikationsbeispiele in der Humanmedizin bzw. Tiermedizin können daher beispielsweise genannt werden:

Die Unterstützung der Wundheilung und Epithelisierung wie z.B. bei Brand, Schürf- und Schnittwunden, bei chronischen Ulzera, Dekubitus und Analfissuren. Weiterhin können die erfindungsgemäßen Verbindungen zur Nachbehandlung bei Hauttransplantationen und operativen Eingriffen bei Zervixerosionen dienen. Die erfindungsgemäßen Verbindungen eignen sich ebenfalls zur Prophylaxe und Therapie von Brustrhagaden, sowie Windelerythemen und Sonnenbrand. Aufgrund ihrer antimikrobiellen Wirkung können die erfindungsgemäßen Verbindungen auch bei der Behandlung von infizierten Wunden eingesetzt werden.

Zur vorliegenden Erfindung gehören pharmazeutische Zubereitungen, wie sie bereits in EP 0 365 915 beschrieben sind, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Bevorzugt sind pharmazeutische Zubereitungen (Formulierungen), die sich für die topische Applikation eignen.

Der Ausdruck "topisch", wie er in der vorliegenden Beschreibung verwendet wird, bezieht sich auf die Verwendung des aktiven Bestandteils, das mit einem geeigneten Trägermaterial verarbeitet und auf die Haut oder Schleimhaut gebracht wird, so daß es die lokale Wirkung entfalten kann. Demgemäß umfassen die topischen Arzneimittel für die externe Anwendung geeignete, pharmazeutische Dosierungsformen, so daß ein direkter Kontakt mit der Haut entsteht. Die topischen Dosierungsformen umfassen Gele, Cremes, Lotionen, Salben, Puder, Aerosole und andere herkömmliche Formen, welche für die direkte Applikation von Arzneimitteln auf die Haut oder Schleimhaut geeignet sind.

Zur vorliegenden Erfindung gehören auch pharmazeutische Zubereitungen in Dosierungseinheiten. Dies bedeutet, daß die Zubereitungen in Form einzelner Teile, z.B. Suppositorien und Ampullen vorliegen, deren Wirkstoffgehalt einen Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Granulate, Suppositorien, Lösungen, Suspensionen und Emulsionen, Pasten, Salben, Gele, Cremes, Lotions, Puder oder Sprays genannt.

Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der Unter (a) bis (i) aufgeführten Stoffe.

Die Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffen enthalten, z.B. Polyethylenglykole, Fette, z.B. Kakaofett und höhere Ester (z.B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Aceton, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein. Zubereitungen für die topische Anwendung können beispielsweise 0,1 bis 10 Gew.-%, vorzugsweise 0,5 bis 5 Gew.-% an therapeutisch wirksamer Verbindung bezogen auf die Gesamtmischung enthalten. Je nach Dosierungsform können jedoch auch höhere oder niedrigere Konzentrationen vorliegen.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal und/oder rektal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

### Beispiele

### A. Wundheilungsaktivität

Die Wirkung der Verbindungen wird auf folgende Weise getestet:
Untersucht wird die Reparatur der Permeabilitätsbarriere der Haut und die Beschleunigung der Wundheilung. Die betreffende Verbindung wird in Form einer 1 %igen Lösung in einem Lösungsmittelgemisch aus 50 Vol.-% Aceton und 50 Vol.-% Propylenglykol appliziert. Als Kontrolle wurde zum Vergleich jeweils das reine Vehikel (50 Vol-% Aceton/50 Vol-% Propylenglykol appliziert.

### a) Reparatur der Permeabilitätsbarriere der Haut

Man untersucht die Reparatur der Permeabilitätsbarriere der Haut nach artifizieller Barrierestörung. Hierzu werden haarlose Mäuse mit Aceton, das mit einem Wattetupfer aufgetragen wird, behandelt, bis sich ein etwa 30-facher Anstieg des transepidermalen Wasserverlustes (TEWL) ergibt. Sofort nach Störung der Barriere wird die betreffende Substanz bzw. das Vehikel (50 Vol.-% Aceton/50 Vol.-% Propylenglykol, 25 µl) aufgetragen. Es werden 7 haarlose Mäuse in jeder Gruppe verwendet. Jeweils zwei Meßstellen werden im behandelten Areal ausgewertet. Die Barrierereparatur wird nach 1, 3, 5 und 24 Stunden gemessen.

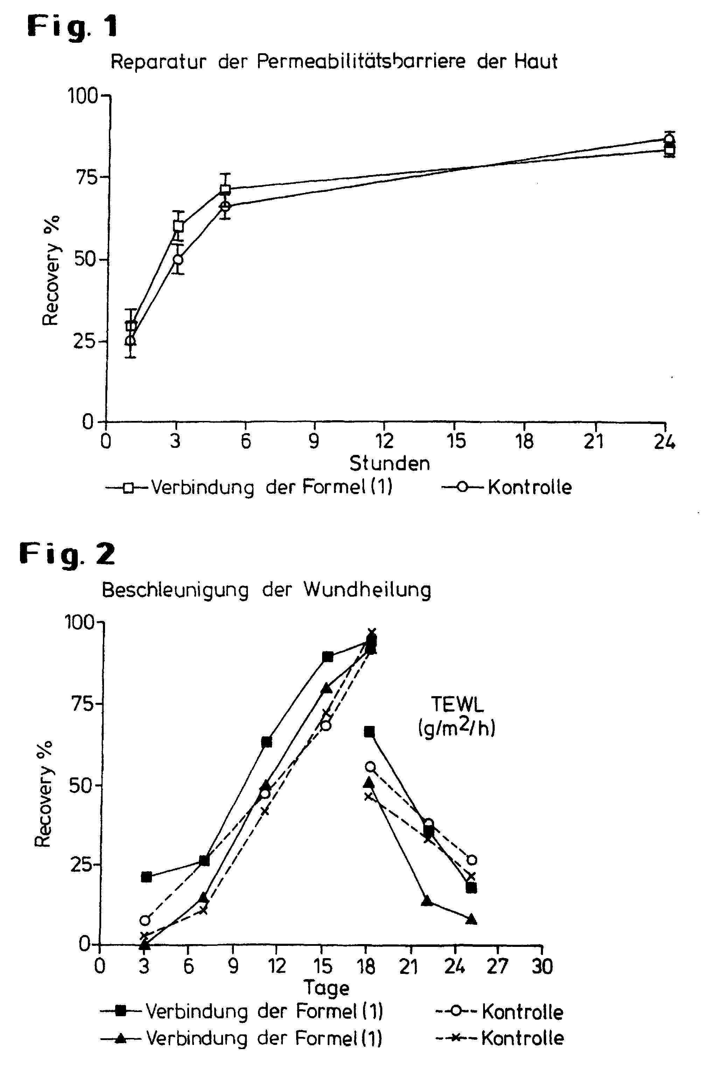

Versuche mit der Verbindung 4-[2-(2,4-Dimethylphenoxy)-phenyl]-2-[2-(1,4,5,6-tetrahydropyrimidinyl)-amino]-thiazol der Formel (1) 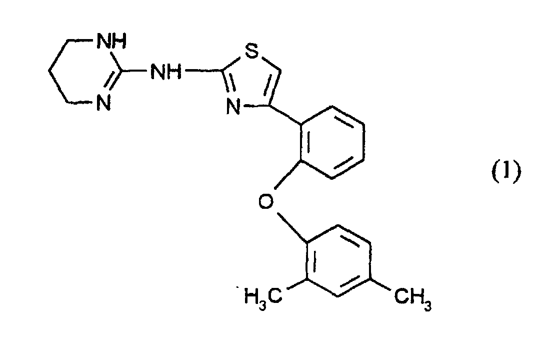 ergaben eine signifikant beschleunigte Reparatur der Permeabilitätsbarriere nach 3 und 5 Stunden (siehe Fig. 1).

### b) Beschleunigung der Wundheilung

Die Wundheilung wird nach der Vorschrift von Mellin et al. (Merck Research Laboratories, USA, J. Invest. Dermatol. 104 (1995), 850 bis 855) untersucht. Hierzu wird an diabetischen haarlosen Mäusen ein runder Hautdefekt mit etwa 2 cm Durchmesser durch Exzision der gesamten Haut herbeigeführt. Direkt danach (Tag 0) und an den Tagen 3 und 7 erfolgt die Applikation von 25 µl einer Lösung der Verbindung in dem obengenannten Lösungsmittel. Zum Vergleich wird auch das reine Lösungsmittel (Vehikel) aufgetragen. Die Wunden werden mit einer semipermeablen Folie (Comfeel plus) für 18 Tage abgedeckt. Die Größe der Wunde wird an den Tagen 0, 3, 7, 11, 15 und 18 bestimmt. An den Tagen 22 und 25 wird der TEWL im ehemaligen Wundgebiet gemessen.

Versuche mit der o.g. Verbindung 4-[2-(2,4-Dimethylphenoxy)-phenyl]-2-[2-(1,4,5,6-tetrahydropyrimidinyl)-amino]-thiazol der Formel (1) 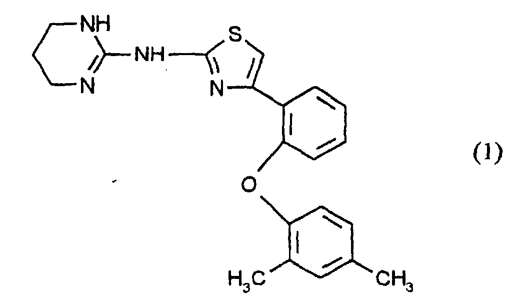 ergaben eine signifikant beschleunigte Wundheilung (siehe Fig. 2).

### B. Formulierungsbeispiele

### Lösungsformulierung

1 g der Verbindung der Formel (1) wird unter Rühren in 99 g eines Lösungsmittelgemischs aus 50 Vol-% Aceton und 50 Vol-% Propylenglykol gelöst.

## Patentansprüche

1. Verwendung von 2-Aminothiazolen der allgemeinen Formel (I) 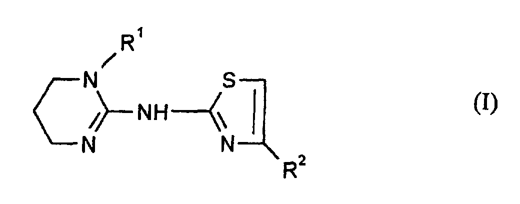 in welcher
R¹ für Wasserstoff oder für geradkettiges oder verzweigtes Alkyl mit 1 bis 4 Kohlenstoffatomen steht und
R² für einen Rest der Formel 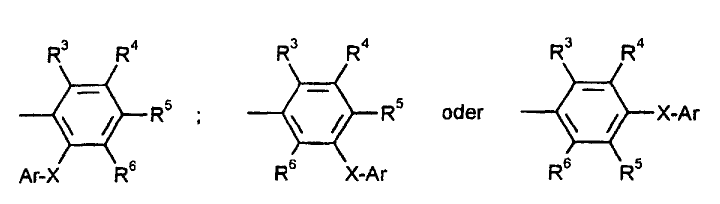 steht,
wobei
R³, R⁴, R⁵ und R⁶ unabhängig voneinander jeweils für Wasserstoff, Fluor, Chlor, Brom, Iod, Nitro, für jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 4 Kohlenstoffatomen in den jeweiligen Alkylteilen oder für jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen - insbesondere Fluor, Chlor, Brom oder Iod - stehen,
X für Sauerstoff, Schwefel, Sulfinyl oder Sulfonyl steht und
Ar für jeweils gegebenenfalls einfach oder mehrfach, gleich oder verschieden substituiertes Phenyl, α-Naphthyl, β-Naphthyl, Tetrahydronaphthyl oder Indanyl steht, wobei als Substituenten jeweils in Frage kommen: Fluor, Chlor, Brom, Iod, jeweils geradkettiges oder verzweigtes Alkyl, Alkoxy, Alkoxycarbonyl, Dialkylamino, Alkylthio, Alkylsulfinyl oder Alkylsulfonyl mit jeweils 1 bis 8 Kohlenstoffatomen in den jeweiligen Alkylteilen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, Cycloalkyl mit 3 bis 7 Kohlenstoffatomen, Phenylalkyl oder Phenoxyalkyl mit jeweils 1 bis 4 Kohlenstoffatomen im geradkettigen oder verzweigten Alkylteil sowie Phenyl oder Phenoxy,
sowie von deren physiologisch verträglichen Säureadditionssalzen,
zur Herstellung von Arzneimitteln zur Verbesserung der Wundheilung.

2. Verwendung gemäß Anspruch 1, **dadurch gekennzeichnet, daß** Verbindungen der Formel (I) eingesetzt werden, bei welchen
R¹ für Wasserstoff, Methyl oder Ethyl steht, und
R² für einen Rest der Formel 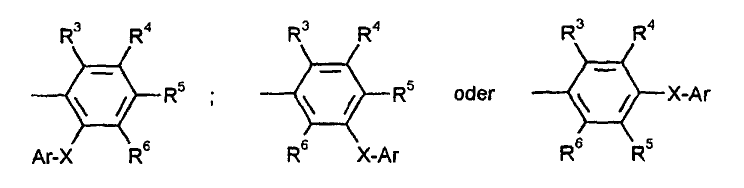 steht,
wobei
R³, R⁴, R⁵ und R⁶ unabhängig voneinander jeweils für Wasserstoff, Fluor, Chlor, Brom, Nitro, Methyl, Ethyl, Methoxy, Ethoxy, Methoxycarbonyl, Ethoxycarbonyl, Dimethylamino, Diethylamino, Methylthio, Ethylthio, Methylsulfinyl, Ethylsulfinyl, Methylsulfonyl oder Ethylsulfonyl oder für Halogenmethyl, Halogenethyl, Halogenmethoxy, Halogenethoxy, Halogenmethylthio, Halogenethylthio, Halogenmethylsulfinyl, Halogenethylsulfinyl, Halogenmethylsulfonyl oder Halogenethylsulfonyl mit jeweils 1 bis 5 gleichen oder verschiedenen Halogenatomen, insbesondere Fluor, Chlor oder Brom stehen,
X für Sauerstoff, Schwefel, Sulfinyl oder Sulfonyl steht und
Ar für jeweils gegebenenfalls ein- bis fünffach gleich oder verschieden substituiertes Phenyl, α-Naphthyl, β-Naphthyl, Tetrahydronaphthyl oder Indanyl steht, wobei als Substituenten jeweils in Frage kommen: Fluor, Chlor, Brom, Nitro, jeweils geradkettiges oder verzweigtes Alkyl, Alkoxy, Alkoxycarbonyl oder Dialkylamino mit jeweils 1 bis 6 Kohlenstoffatomen in den jeweiligen Alkylteilen, jeweils geradkettiges oder verzweigtes Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, insbesondere Fluor, Chlor oder Brom, Cyclohexyl mit 3 bis 6 Kohlenstoffatomen, Phenylalkyl oder Phenoxyalkyl mit jeweils 1 bis 3 Kohlenstoffatomen im geradkettiges oder verzweigten Alkylteil sowie Phenyl oder Phenoxy.

3. Verwendung gemäß Anspruch 1, **dadurch gekennzeichnet, daß** Verbindungen der Formel (I) eingesetzt werden, bei welchen
R¹ für Wasserstoff oder Methyl steht und
R² für einen Rest 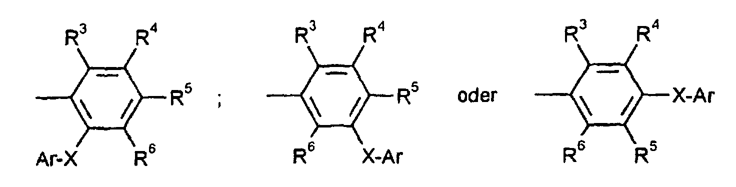 steht,
wobei
R³, R⁴, R⁵ und R⁶ unabhängig voneinander jeweils für Wasserstoff, Fluor, Chlor, Nitro, Methyl, Methoxy, Ethoxycarbonyl, Methoxycarbonyl, Dimethylamino, Methylthio, Trifluormethyl, Trifluormethoxy, Trifluormethylthio, Trifluormethylsulfinyl oder Trifluormethylsulfonyl stehen,
X für Sauerstoff oder Schwefel stehen und
Ar für jeweils gegebenenfalls ein- bis vierfach, gleich oder verschieden substituiertes Phenyl, α-Naphthyl, β-Naphthyl, Tetrahydronaphthyl oder Indanyl steht, wobei als Substituenten jeweils in Frage kommen: Fluor, Chlor, Brom, Nitro, Methyl, Ethyl, n- oder i-Propyl, n-, i-, s- oder t-Butyl, Methoxy, Ethoxy, n- oder i-Propoxy, Methoxycarbonyl, Ethoxycarbonyl, Dimethylamino, Diethylamino, Methylthio, Ethylthio, Methylsulfinyl, Methylsulfonyl, Trifluormethyl, Trifluormethoxy, Trifluormethylthio, Trifluormethylsulfinyl, Trifluormethylsulfonyl, Cyclopentyl, Cyclohexyl, Benzyl, Phenylethyl, Phenylpropyl, Phenoxymethyl, Phenyl oder Phenoxy.

4. Verwendung gemäß Anspruch 1, **dadurch gekennzeichnet, daß** Verbindungen der Formel (I) eingesetzt werden, bei welchen
R¹ für Wasserstoff steht und
R² für einen Rest der Formel 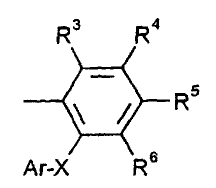 steht,
wobei
R³, R⁴, R⁵ und R⁶ unabhängig voneinander jeweils für Wasserstoff, Methyl oder Nitro stehen,
X für Sauerstoff steht und
Ar für gegebenenfalls ein- bis dreifach, gleich oder verschieden substituiertes Phenyl steht, wobei als Substituenten insbesondere in Frage kommen: Nitro, Methyl, Ethyl, n- oder i-Propyl, n-, i-, s- oder t-Butyl, Methoxy, Methylthio, Cyclopentyl, Cyclohexyl, Phenyl oder Phenoxy.

## Claims

1. Use of 2-aminothiazoles of the general formula (I) 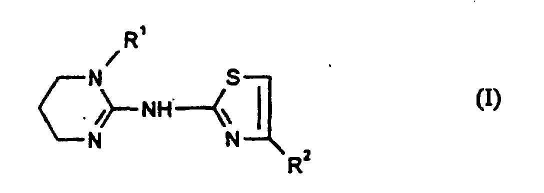 in which
R¹ represents hydrogen, or represents straight-chain or branched alkyl having 1 to 4 carbon atoms and
R² represents a radical of the formula 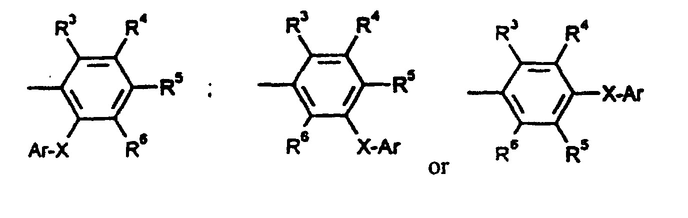 wherein
R³, R⁴, R⁵ and R⁶ independently of one another each represent hydrogen, fluorine, chlorine, bromine, iodine or nitro, or represent in each case straight-chain or branched alkyl, alkoxy, alkoxycarbonyl, dialkylamino, alkylthio, alkylsulphinyl or alkylsulphonyl having in each case 1 to 4 carbon atoms in the particular alkyl parts, or represent in each case straight-chain or branched halogenoalkyl, halogenoalkoxy, halogenoalkylthio, halogenoalkylsulphinyl or halogenoalkylsulphonyl having in each case 1 to 4 carbon atoms and 1 to 9 identical or different halogen atoms - in particular fluorine, chlorine, bromine or iodine,
X represents oxygen, sulphur, sulphinyl or sulphonyl and
Ar represents phenyl, α-naphthyl, β-naphthyl, tetrahydronaphthyl or indanyl which are in each case optionally substituted once or several times in an identical or different manner, possible substituents in each case being: fluorine, chlorine, bromine, iodine, in each case straight-chain or branched alkyl, alkoxy, alkoxycarbonyl, dialkylamino, alkylthio, alkylsulphinyl or alkylsulphonyl having in each case 1 to 8 carbon atoms in the particular alkyl parts, in each case straight-chain or branched halogenoalkyl, halogenoalkoxy, halogenoalkylthio, halogenoalkylsulphinyl or halogenoalkylsulphonyl having in each case 1 to 4 carbon atoms and 1 to 9 identical or different halogen atoms, cycloalkyl having 3 to 7 carbon atoms, phenylalkyl or phenoxyalkyl having in each case 1 to 4 carbon atoms in the straight-chain or branched alkyl part, and phenyl or phenoxy,
and of physiologically tolerated acid addition salts thereof for preparing medicaments
for improving wound healing.

2. Use according to Claim 1, **characterized in that** compounds of the formula (I) in which
R¹ represents hydrogen, methyl or ethyl and
R² represents a radical of the formula 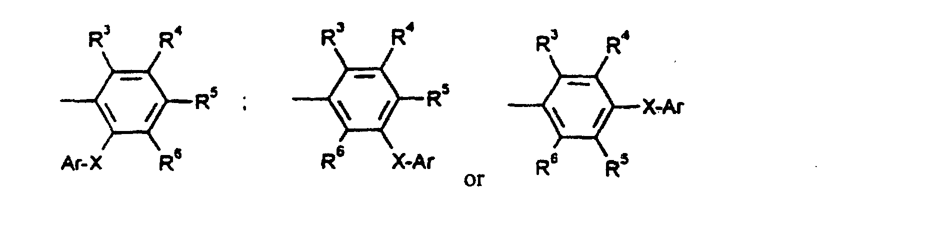 wherein
R³, R⁴, R⁵ and R⁶ independently of one another each represent hydrogen, fluorine, chlorine, bromine, nitro, methyl, ethyl, methoxy, ethoxy, methoxycarbonyl, ethoxycarbonyl, dimethylamino, diethylamino, methylthio, ethylthio, methylsulphinyl, ethylsulphinyl, methylsulphonyl or ethylsulphonyl or represent halogenomethyl, halogenoethyl, halogenomethoxy, halogenoethoxy, halogenomethylthio, halogenoethylthio, halogenomethylsulphinyl, halogenoethylsulphinyl, halogenomethylsulphonyl or halogenoethylsulphonyl having in each case 1 to 5 identical or different halogen atoms, in particular fluorine, chlorine or bromine,
X represents oxygen, sulphur, sulphinyl or sulphonyl and
Ar represents phenyl, α-naphthyl, β-naphthyl, tetrahydronaphthyl or indanyl, in each case optionally substituted once to five times in an identical or different manner, possible substituents in each case being: fluorine, chlorine, bromine, nitro, in each case straight-chain or branched alkyl, alkoxy, alkoxycarbonyl or dialkylamino having in each case 1 to 6 carbon atoms in the particular alkyl parts, in each case straight-chain or branched alkylthio, alkylsulphinyl or alkylsulphonyl having in each case 1 to 3 carbon atoms, in each case straight-chain or branched halogenoalkyl, halogenoalkoxy, halogenoalkylthio, halogenoalkylsulphinyl or halogenoalkylsulphonyl having in each case 1 to 3 carbon atoms and 1 to 7 identical or different halogen atoms, in particular fluorine, chlorine of bromine, cyclohexyl having 3 to 6 carbon atoms, phenylalkyl or phenoxyalkyl having in each case 1 to 3 carbon atoms in the straight-chain or branched alkyl part, and phenyl or phenoxy,
are employed.

3. Use according to Claim 1, **characterized in that** compounds of the formula (I) in which
R¹ represents hydrogen or methyl and
R² represents a radical of the formulae 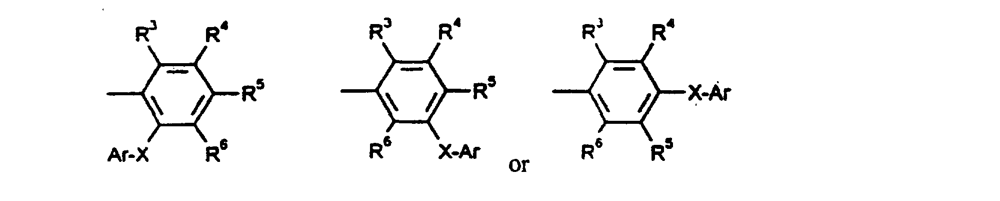 wherein
R³, R⁴, R⁵ and R⁶ independently of one another each represent hydrogen, fluorine, chlorine, nitro, methyl, methoxy, ethoxycarbonyl, methoxycarbonyl, dimethylamino, methylthio, trifluoromethyl, trifluoromethoxy, trifluoromethylthio, trifluoromethylsulphinyl or trifluoromethyl-sulphonyl,
X represents oxygen or sulphur and
Ar represents phenyl, α-naphthyl, β-naphthyl, tetrahydronaphthyl or indanyl, in each case optionally substituted once to four times in an identical or different manner, possible substituents in each case being: fluorine, chlorine, bromine, nitro, methyl, ethyl, n- or i-propyl, n-, i-, s- or t-butyl, methoxy, ethoxy, n- or i-propoxy, methoxycarbonyl, ethoxycarbonyl, dimethylamino, diethylamino, methylthio, ethylthio, methylsulphinyl, methylsulphonyl, trifluoromethyl, trifluoromethoxy, trifluoromethylthio, trifluoromethylsulphinyl, trifluoromethylsulphonyl, cyclopentyl, cyclohexyl, benzyl, phenylethyl, phenylpropyl, phenoxymethyl, phenyl or phenoxy,
are employed.

4. Use according to Claim 1, **characterized in that** compounds of the formula (I) in which
R¹ represents hydrogen and
R² represents a radical of the formula 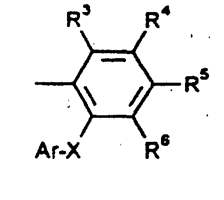 wherein
R³, R⁴, R⁵ and R⁶ independently of one another each represent hydrogen, methyl or nitro,
X represents oxygen and
Ar represents phenyl which is optionally substituted once to three times by identical or different substituents, possible substituents being, in particular: nitro, methyl, ethyl, n- or i-propyl, n-, i-, s- or t-butyl, methoxy, methylthio, cyclopentyl, cyclohexyl, phenyl or phenoxy,
are employed.

## Revendications

1. Utilisation de 2-aminothiazoles de formule générale (I) 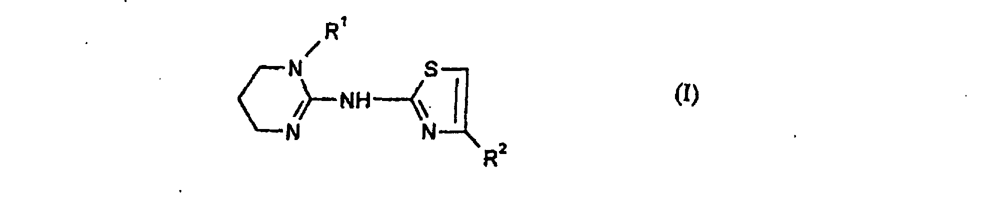 où
R¹ représente l'hydrogène ou un alkyle linéaire ou ramifié ayant 1 à 4 atomes de carbone et
R² représente un résidu de formule 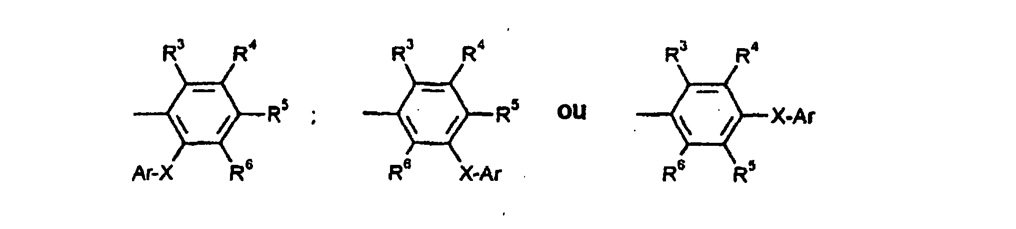 où
R³, R⁴, R⁵ et R⁶ représentent indépendamment les uns des autres l'hydrogène, le fluor, le chlore, le brome, l'iode, le nitro, un alkyle, alcoxy, alcoxycarbonyle, dialkylamino, alkylthio, alkylsulfinyle ou alkylsulfonyle linéaires ou ramifiés ayant chacun 1 à 4 atomes de carbone dans la partie alkyle ou un halogénoalkyle, halogénoalcoxy, halogénoalkylthio, halogénoalkylsulfinyle ou halogénoalkylsulfonyle linéaires ou ramifiés ayant chacun 1 à 4 atomes de carbone et 1 à 9 atomes d'halogènes identiques ou différents - en particulier fluor, chlore, brome ou iode,
X représente l'oxygène, le soufre, le sulfinyle ou le sulfonyle et
Ar représente un phényle, α-naphtyle, β-naphtyle, tétrahydronaphtyle ou indanyle substitués de façon identique ou différente le cas échéant une ou plusieurs fois, étant envisagés comme substituants : le fluor, le chlore, le brome, l'iode, un alkyle, alcoxy, alcoxycarbonyle, dialkylamino, alkylthio, alkylsulfinyle ou alkylsulfonyle linéaires ou ramifiés ayant chacun 1 à 8 atomes de carbone dans la partie alkyle, un halogénoalkyle, halogénoalcoxy, halogénoalkylthio, halogénoalkylsulfinyle ou halogénoalkylsulfonyle linéaires ou ramifiés ayant chacun 1 à 4 atomes de carbone et 1 à 9 atomes d'halogènes identiques ou différents, cycloalkyle ayant 3 à 7 atomes de carbone, phénylalkyle ou phénoxyalkyle ayant chacun 1 à 4 atomes de carbone dans la partie alkyle linéaire ou ramifiée, ainsi que phényle ou phénoxy,
ainsi que leurs sels d'addition d'acide physiologiquement compatibles,
pour la préparation de médicaments destinés à améliorer la cicatrisation de plaies.

2. Utilisation selon la revendication 1 **caractérisée en ce que** sont utilisés des composés de formule (I) où
R¹ représente l'hydrogène, le méthyle ou l'éthyle et
R² représente un résidu de formule 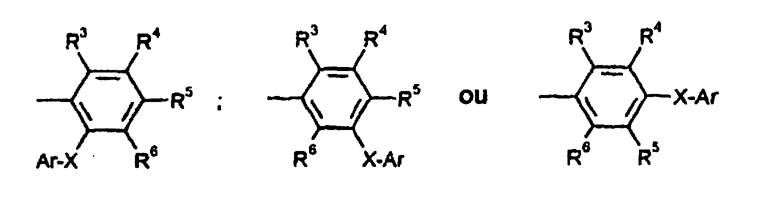 où
R³, R⁴, R⁵ et R⁶ représentent indépendamment les uns des autres l'hydrogène, le fluor, le chlore, le brome, le nitro, le méthyle, l'éthyle, le méthoxy, l'éthoxy, le méthoxycarbonyle, l'éthoxycarbonyle, le diméthylamino, le diéthylamino, le méthylthio, l'éthylthio, le méthylsulfinyle, l'éthylsulfinyle, le méthylsulfonyle ou l'éthylsulfonyle ou un halogénométhyle, halogénoéthyle, halogénométhoxy, halogénoéthoxy, halogénométhylthio, halogénoéthylthio, halogénométhylsulfinyle, halogénoéthylsulfinyle, halogénométhylsulfonyle ou halogénoéthylsulfonyle ayant chacun 1 à 5 atomes d'halogènes identiques ou différents, en particulier le fluor, le chlore ou le brome,
X représente l'oxygène, le soufre, le sulfinyle ou le sulfonyle et
Ar représente un phényle, α-naphtyle, β-naphtyle, tétrahydronaphtyle ou indanyle substitués de façon identique ou différente le cas échéant une à 5 fois, étant envisagés comme substituants : fluor, chlore, brome, nitro, alkyle, alcoxy, alcoxycarbonyle ou dialkylamino linéaires ou ramifiés ayant chacun 1 à 6 atomes de carbone dans la partie alkyle, alkylthio, alkylsulfinyle ou alkylsulfonyle linéaires ou ramifiés ayant chacun 1 à 3 atomes de carbone, halogénoalkyle, halogénoalcoxy, halogénoalkylthio, halogénoalkylsulfinyle ou halogénoalkylsulfonyle linéaires ou ramifiés ayant chacun 1 à 3 atomes de carbone et 1 à 7 atomes d'halogènes identiques ou différents, en particulier fluor, chlore ou brome, cyclohexyle ayant 3 à 6 atomes de carbone, phénylalkyle ou phénoxyalkyle ayant chacun 1 à 3 atomes de carbone dans la partie alkyle linéaire ou ramifiée ainsi que phényle ou phénoxy.

3. Utilisation selon la revendication 1 **caractérisée en ce que** sont utilisés des composés de formule (I) où
R¹ représente l'hydrogène ou le méthyle et
R² représente un résidu 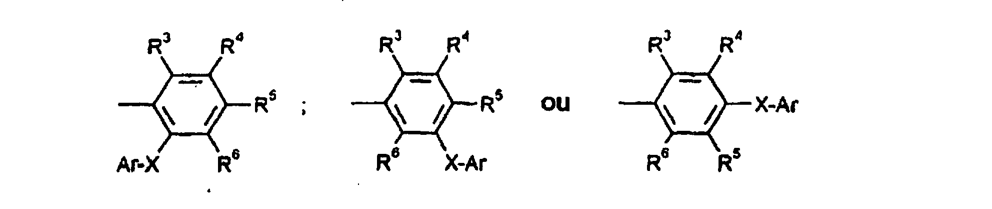 où
R³, R⁴, R⁵ et R⁶ représentent indépendamment les uns des autres l'hydrogène, le fluor, le chlore, le nitro, le méthyle, le méthoxy, l'éthoxycarbonyle, le méthoxycarbonyle, le diméthylamino, le méthylthio, le trifluorométhyle, le trifluorométhoxy, le trifluorométhylthio, le trifluorométhylsulfinyle ou le trifluorométhylsulfonyle,
X représente l'hydrogène ou le soufre et
Ar représente un phényle, α-naphtyle, β-naphtyle, tétrahydronaphtyle ou indanyle substitués de façon identique ou différente le cas échéant une à 4 fois, étant envisagés comme substituants : fluor, chlore, brome, nitro, méthyle, éthyle, n- ou i-propyle, n-, i-, s- ou t-butyle, méthoxy, éthoxy, n- ou i-propoxy, méthoxycarbonyle, éthoxycarbonyle, diméthylamino, diéthylamino, méthylthio, éthylthio, méthylsulfinyle, méthylsulfonyle, trifluorométhyle, trifluorométhoxy, trifluorométhylthio, trifluorométhylsulfinyle, trifluorométhylsulfonyle, cyclopentyle, cyclohexyle, benzyle, phényléthyle, phényl-propyle, phénoxyméthyle, phényle ou phénoxy.

4. Utilisation selon la revendication 1 **caractérisée en ce que** sont utilisés des composés de formule (I) où
R¹ représente l'hydrogène et
R² représente un résidu de formule 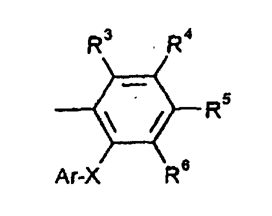 où
R³, R⁴, R⁵ et R⁶ représentent indépendamment les uns des autres l'hydrogène, le méthyle ou le nitro,
X représente l'oxygène et
Ar représente un phényle substitué de façon identique ou différente le cas échéant 1 à 3 fois, étant en particulier envisagés comme substituants : nitro, méthyle, éthyle, n- ou i-propyle, n-, i-, s- ou t-butyle, méthoxy, méthylthio, cyclopentyle, cyclohexyle, phényle ou phénoxy.
